# DEMANDE DE BREVET EUROPEEN

(11) **EP 2 638 880 A1**
(43) Date de publication de la demande: **18.09.2013**
(21) Numéro de dépôt: 12290090.5
(22) Date de dépôt: 16.03.2012
(51) Int. Cl.: A61F 2/44

(54) **Cage intervertébrale**

(71) Demandeur: Razian, Hassan, 94240 L'Hay les Roses (FR)
(72) Inventeur: Razian, Hassan, 94240 L'Hay les Roses (FR)
(74) Mandataire: Flavenot, Bernard

(57) **Abrégé**

La présente invention concerne les cages intervertébrales.

La cage selon l'invention est caractérisée par le fait qu'elle comporte un corps 10 comportant deux faces opposées 11, 12 et une paroi latérale 13, une cavité 15 débouchant sur les faces 11, 12, une patte 21, et des moyens 30 pour monter la patte 21 en rotation dans la cavité 15 autour d'un axe 16 entre deux positions, ces moyens 30 comportant un arbre de rotation 31 solidaire de la première patte 21, et un orifice traversant 32 réalisé dans le corps 10, entre la cavité 15 et la paroi latérale 13, et centré sur l'axe de rotation 16, l'arbre 31 étant monté en rotation dans cet orifice 32 de façon que son axe soit sensiblement confondu avec l'axe 16, et une percée traversante cylindrique 34 réalisée dans l'arbre 31 de façon que son axe soit sensiblement confondu avec l'axe 16, la section transversale 35 de cette percée 34 étant polygonale.

Application aux cages intervertébrales particulièrement avantageuse, mais non exclusivement, pour le traitement du rachis dégénératif.

## Description

La présente invention concerne les cages intervertébrales trouvant une application particulièrement avantageuse, mais non exclusivement, pour le traitement du rachis dégénératif.

Une cage intervertébrale, notamment pour le traitement du rachis dégénératif, est déjà connue. Elle comporte essentiellement une entretoise en forme de disque comprenant deux faces de base opposées sensiblement planes et parallèles et une paroi latérale reliant les deux faces de base. Cette entretoise est apte à être disposée entre les faces en regard des deux corps vertébraux respectivement de deux vertèbres consécutives, en remplacement du disque endommagé situé entre ces deux vertèbres, les deux faces de base de l'entretoise étant placées au contact des corps vertébraux. L'entretoise comporte en outre une cavité ouverte dans laquelle il est possible de placer un greffon osseux ou analogue dans le but de souder entre eux les deux corps vertébraux par ostéosynthèse.

Bien que cette réalisation donne généralement de bons résultats, elle peut, dans des circonstances très exceptionnelles, ne pas permettre à la cage intervertébrale de remplir le rôle pour lequel elle était prévue.

En effet, dans les premiers temps suivant l'implantation de la cage, on constate que, lors de certains mouvements de relativement grande amplitude de la colonne vertébrale, par exemple en ce qui concerne les vertèbres cervicales quand la tête se penche très en arrière, il peut se créer un espace non négligeable entre les faces de base de l'entretoise et les vertèbres, par lequel le greffon ou une partie de celui-ci peut s'échapper de la cavité dans laquelle il était placé. Si c'est le cas, le greffon ne peut plus alors jouer correctement son rôle. Ou bien l'absence d'un ancrage efficace engendre des charges de cisaillement qui ne peuvent immobiliser la cage en post opératoire, ce qui rend nécessaire une intervention chirurgicale de révision.

Pour tenter de pallier les inconvénients mentionnés ci-dessus, il a été conçu des cages intervertébrales comportant une patte d'ancrage pivotant sur une face de l'entretoise, comme les cages décrites dans les US-A-5 683 394 et FR-A-2930426. Ces réalisations donnent théoriquement de bons résultats mais présentent parfois des insuffisances, notamment quant au maintien de la patte d'ancrage dans sa position de blocage de l'entretoise.

Il a même été proposé une cage intervertébrale avec deux pattes ou lamelles, comme celle décrite et illustrée dans le EP 1 674 053. Cependant, cette réalisation n'est pas pratique à implanter et, en outre, ne permet pas d'utiliser une quantité suffisante de greffons pour favoriser et accélérer l'ostéosynthèse entre les deux vertèbres.

Aussi, la présente invention a-t-elle pour but de réaliser une cage intervertébrale qui pallie les inconvénients mentionnés ci-dessus des cages intervertébrales décrites succinctement dans ce préambule, et dont la structure permet en plus un très bon ancrage, et fiable, de la cage dans les vertèbres entre lesquelles elle doit être implantée.

Plus précisément, la présente invention a pour objet une cage intervertébrale comportant les caractéristiques énoncées dans la revendication 1 annexée.

D'autres caractéristiques et avantages de la présente invention apparaîtront au cours de la description suivante donnée en regard des dessins annexés à titre illustratif mais nullement limitatif, dans lesquels :
La figure 1 est une vue de dessus d'une coupe longitudinale d'un mode de réalisation de la cage intervertébrale selon l'invention, la coupe selon cette figure étant référencée 1-1 sur la figure 2 définie ci-après,
La figure 2 est une vue de côté de la cage intervertébrale selon l'invention dans le même mode de réalisation que celui représenté sur la figure 1, et
La figure 3 est une vue partielle schématique d'un autre mode de réalisation de la cage selon l'invention, perfectionné par rapport au mode de réalisation selon les figures 1 et 2.

Il est tout d'abord précisé que, sur les figures, les mêmes références désignent les mêmes éléments, quelle que soit la figure sur laquelle elles apparaissent et quelle que soit la forme de représentation de ces éléments.

Il est aussi précisé que, dans la présente description, si l'adverbe "sensiblement" est associé à un qualificatif d'un moyen donné, ce qualificatif doit être compris au sens strict ou approché.

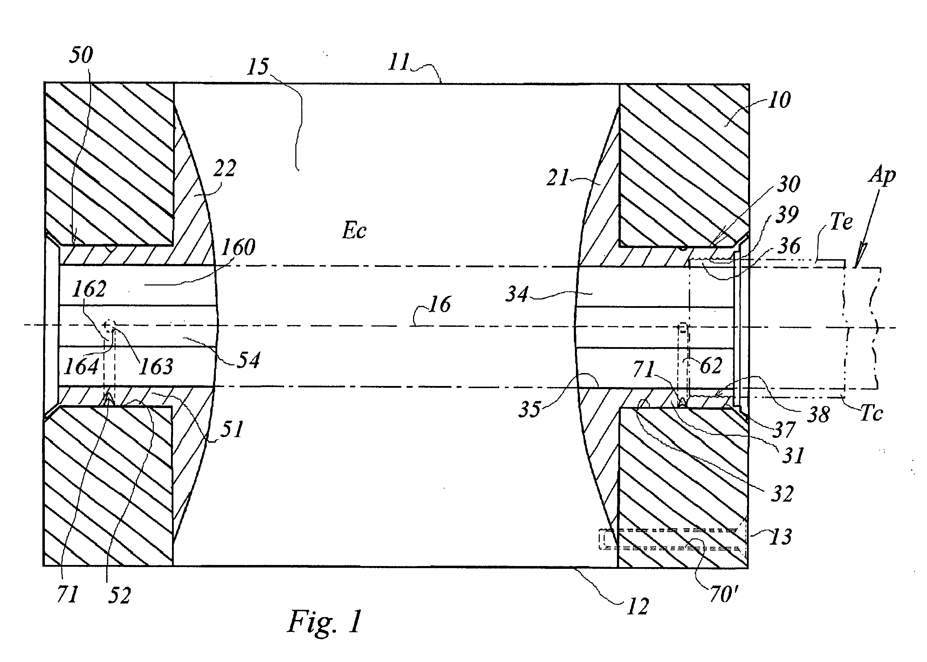

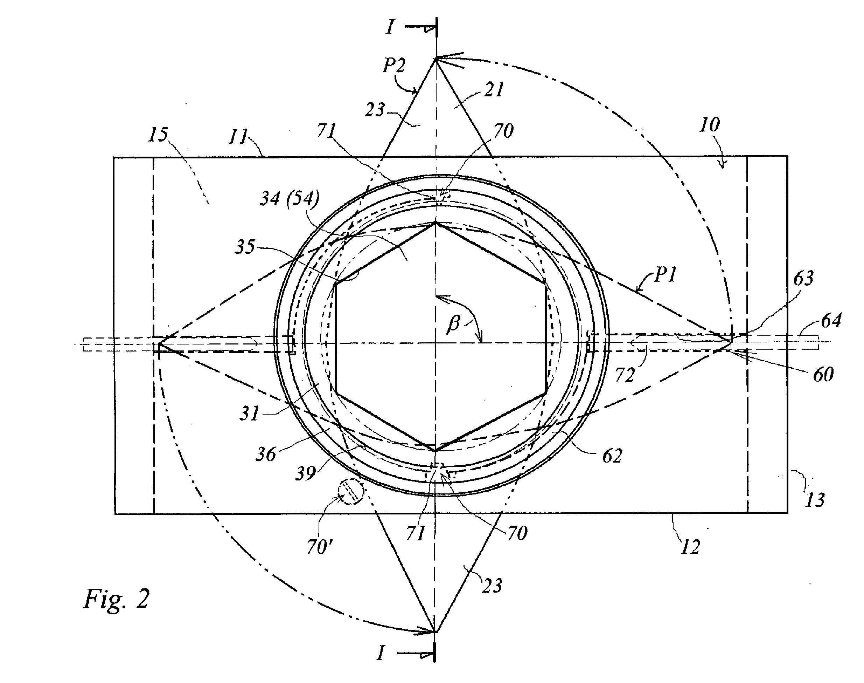

En référence aux deux figures 1 et 2 annexées, la présente invention concerne une cage intervertébrale qui trouve une application particulièrement avantageuse, mais non exclusivement, pour le traitement du rachis dégénératif.

La cage comprend un corps 10 apte à être interposé entre deux vertèbres consécutives et comportant deux faces de base 11, 12 opposées sensiblement planes et parallèles, et une paroi latérale 13 reliant les deux faces de base. Ce corps est réalisé, par exemple en titane ou tout autre matériau biocompatible, ou biodégradable, par exemple en PEEK ou analogue.

La cage comporte aussi au moins une cavité 15 réalisée dans le corps 10 en débouchant sur ses deux faces de base 11, 12, une première patte 21, se présentant par exemple sous la forme d'une lame ou analogue, et des moyens 30 pour monter cette première patte 21 en rotation dans la cavité 15 autour d'un axe de rotation 16 sensiblement parallèle au plan des faces de base 11, 12 entre au moins deux première et seconde positions, figure 2.

La première position est celle dans laquelle la première patte est entièrement contenue dans la cavité 15, figure 1, et la seconde position étant celle dans laquelle une partie 23 de l'une au moins des extrémités de la première patte émerge de la cavité pour pouvoir s'ancrer dans le corps osseux des vertèbres.

Les moyens 30 pour monter en rotation la première patte 21 comportent un premier arbre de rotation 31 solidaire de la première patte et un premier orifice traversant 32 réalisé dans le corps 10, entre la cavité 15 et la paroi latérale 13, et en étant sensiblement centré sur l'axe de rotation 16, le premier arbre de rotation 31 étant monté en rotation dans ce premier orifice traversant 32 de façon que son axe soit sensiblement confondu avec l'axe de rotation 16.

Selon une caractéristique importante de l'invention, ces moyens 30 pour monter en rotation la première patte 21 comportent en outre une première percée traversante cylindrique 34 réalisée dans le premier arbre 31 de façon que son axe soit sensiblement confondu avec l'axe de rotation 16, la section transversale 35 de cette première percée traversante 34 étant polygonale. Cette caractéristique est très avantageuse, notamment dans le cas où la cage comporte deux pattes, comme illustré sur les deux figures.

Selon une autre caractéristique importante de l'invention, ces moyens 30 comportent un lamage 36 réalisé dans l'extrémité 37 du premier arbre 31 qui débouche sur la paroi latérale 13 du corps 10, ce lamage entourant la première percée traversante 34 et étant sensiblement centré sur l'axe de rotation 16, et des moyens de fixation 38 d'un outil de préhension réalisés en coopération avec le lamage 36. Un tel outil de préhension sera décrit ci-après dans le cadre de la description de l'utilisation de cette cage intervertébrale.

A titre d'exemple avantageux, ces moyens de fixation 38 d'un outil de préhension sont constitués d'un taraudage 39 réalisé dans la paroi cylindrique latérale du lamage 36.

Comme évoqué ci-dessus, de façon tout à fait avantageuse, la cage comporte en outre une seconde patte 22, des moyens 50 pour monter la seconde patte en rotation dans la cavité 15 autour du même axe de rotation 16 que défini ci-dessus, entre au moins deux première et seconde positions, la première position étant celle dans laquelle cette seconde patte est entièrement contenue dans la cavité 15 et la seconde position étant celle dans laquelle une partie de l'une au moins des extrémités de la seconde patte 22 émerge de la cavité, exactement comme la première patte 21 définie ci-dessus, du moins d'un point de vue fonctionnel.

Les moyens 50 pour monter en rotation la seconde patte 22 comportent un second arbre de rotation 51 solidaire de la seconde patte, et un second orifice traversant cylindrique de révolution 52 réalisé dans le corps 10, entre la cavité 15 et la paroi latérale 13 du corps, ce second orifice traversant 52 étant sensiblement centré sur le même axe de rotation 16, le second arbre 51 étant alors monté en rotation dans ce second orifice traversant 52 de façon que son axe soit sensiblement confondu avec cet axe de rotation 16.

Comme pour la patte 21, ces derniers moyens 50 comportent une seconde percée 54 réalisée dans le second arbre 51 de façon que son axe soit sensiblement confondu avec l'axe de rotation 16 et qu'elle débouche au moins dans la cavité 15, la section transversale de cette seconde percée 54 étant polygonale et ayant une valeur au plus égale à celle de la première percée traversante 34.

Il est précisé que cette seconde percé 54 doit déboucher dans la cavité 15, mais pas obligatoirement sur la paroi latérale 13 du corps 10, bien que, sur la figure 1, elle ait été représentée y débouchant. Le mode de réalisation illustré est avantageux car, de ce fait, les deux ensembles patte/arbre peuvent être identiques avant que soit réalisé le lamage 36 dans l'arbre correspondant à la première patte, ce qui réduit le coût de fabrication de la cage.

Selon une réalisation préférée, les sections transversales respectivement des première et seconde percées 34, 54 sont identiques, optionnellement de forme polygonale "six pans".

Avantageusement, la cage comporte en outre des moyens 60 pour limiter l'amplitude de la rotation de la première patte 21 par rapport au corps 10 qui, selon une réalisation préférée, comportent au moins une rainure circulaire 62 réalisée dans la paroi extérieure du premier arbre 31 en étant inscrite dans un angle β défini par les première et seconde positions de la première patte, en l'espèce généralement de l'ordre de quatre-vingt-dix degrés, comme représenté sur la figure 2.

Ces moyens 60 comportent en outre un trou 63 percé dans le corps 10 et débouchant en regard de la rainure circulaire 62 et sur la paroi latérale 13 du corps, et une goupille 64 disposée dans le trou 63 et apte à plonger dans la rainure circulaire 62.

Toujours de façon préférentielle, la cage comporte en outre des moyens 70, 70' pour bloquer la première patte 21 quand elle est dans sa seconde position P2. Ces moyens peuvent être de différentes formes et structures. Par exemple comme illustré en 70' sur les figures, ils peuvent être constitués par une vis qui traverse le corps 10 pour déboucher dans la cavité et se placer derrière la patte quand elle est dans sa seconde position.

Cependant, comme illustré en 70 sur les figures, ces moyens 70 pour bloquer la première patte 21 quand elle arrive dans sa seconde position P2 sont très avantageusement constitués par un logement 71 réalisé à l'extrémité de la rainure circulaire 62 située en regard du trou 63 quand la première patte 21 est dans sa seconde position, ce logement 71 étant apte à recevoir l'extrémité pénétrante 72 de la goupille 64 par un enfoncement supplémentaire de celle-ci dans le trou 63.

De façon préférentielle, la cage comporte aussi des moyens 160 pour limiter l'amplitude de la rotation de la seconde patte 22, constitués par au moins une autre rainure circulaire 162 réalisée dans la paroi extérieure du second arbre 51 et inscrite dans un angle β' (qui est généralement le même que l'angle β défini ci-avant) défini par les première et seconde positions de la seconde patte 22, un autre trou 163 percé dans le corps et débouchant en regard de cette autre rainure circulaire et sur la paroi latérale 13 du corps, et une autre goupille 164 disposée dans cet autre trou et apte à plonger dans l'autre rainure circulaire.

De même que pour la première patte 21, la cage comporte des moyens pour bloquer la seconde patte 22 quand elle arrive dans sa seconde position, constitués de préférence par un autre logement réalisé à l'extrémité de l'autre rainure circulaire située en regard de l'autre trou 163 quand la seconde patte 22 est dans sa seconde position, cet autre logement étant apte à recevoir l'extrémité pénétrante de l'autre goupille 164 par un enfoncement supplémentaire de celle-ci dans l'autre trou 163.

Selon une autre caractéristique de l'invention, la cage intervertébrale comporte des moyens pour sertir au moins l'un des deux arbres 31, 51, au niveau de la sortie de l'orifice 32, 52 dans lequel il se trouve, sur la paroi latérale 13 du corps 10 pour fixer l'ensemble patte/arbre sur le corps 10, tout en permettant la rotation de l'arbre dans l'orifice cylindrique de révolution correspondant. Ces derniers moyens sont bien connus en eux-mêmes et ne seront pas plus amplement décrits ici car étant du domaine de l'homme du métier.

Il est ajouté que la cage intervertébrale telle que décrite ci-dessus est plus particulièrement adaptée pour son implantation selon la technique dite "par voie antérieure".

Cependant, de façon optionnelle, pour qu'elle puisse être utilisée pour une implantation par une autre voie, les moyens 30 pour monter en rotation au moins la première patte 21 (et avantageusement les deux pattes 21, 22 en même temps) comportent en outre des moyens 400 pour faire pivoter la patte par l'une de ses extrémités.

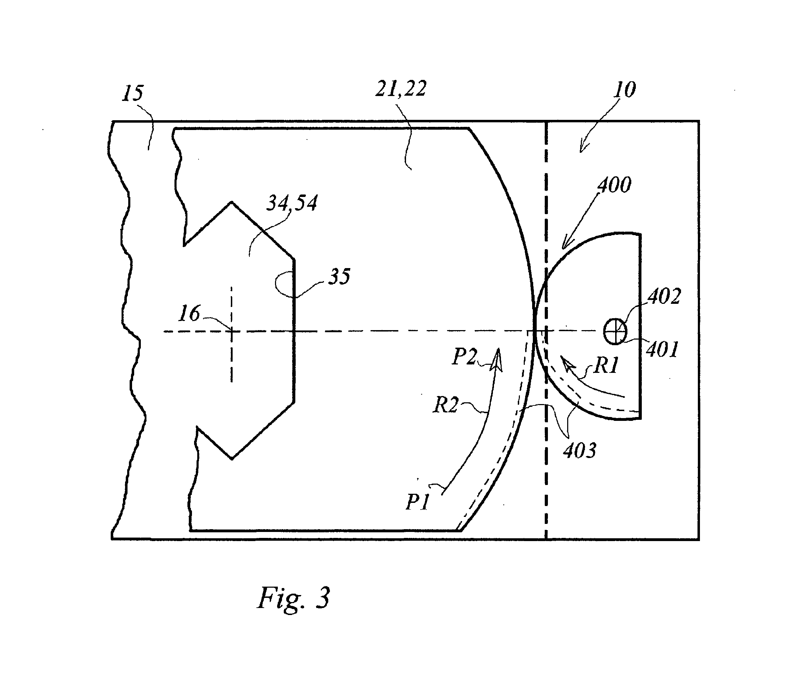

Ces derniers moyens 400, figure 3 qui représente la cage lorsque les pattes 21, 22 sont dans leur première position P1, peuvent être constitués, par exemple, par une tige 401 montée rotative latéralement dans le corps 10 selon un axe 402 parallèle à l'axe de rotation 16 défini ci-avant, et émergeant du corps par au moins l'une de ses extrémités, et par des moyens pour coupler cette tige 401 avec une extrémité de chacune des deux pattes 21, 22. Ces moyens de couplage sont par exemple du type "à crémaillère" et comportent des crans 403 (schématiquement illustrés) ou analogues de façon que, lorsque la tige 401 est pivotée R1 autour de son axe 402, elle entraîne la rotation R2 des deux pattes 21, 22 par leur extrémité, dans une amplitude suffisante pour qu'elles émergent toutes les deux au moins partiellement de la cavité 15 et prennent leur seconde position P2.

La cage intervertébrale selon l'invention telle que décrite ci-dessus s'utilise de la façon suivante :
Il est tout d'abord mentionné que la cage telle que décrite ci-dessus est fabriquée en usine, et arrive généralement chez le Praticien chirurgien sous blister et stérilisée, les pattes 21, 22, comme des lames ou analogues, étant disposées dans leur première position P1. Quant aux deux percées 34, 54, elles sont réalisées de façon que leurs sections transversales soient sur une même enveloppe cylindrique Ec, figure 1.

Le Praticien a aussi à sa disposition un ancillaire de pose Ap essentiellement constitué d'un tube extérieur Te dont une extrémité a sa surface extérieure filetée de façon complémentaire au taraudage 39 du lamage 36, et d'un tube central intérieur Tc (par exemple du type "clef hexagonale") ayant la même section latérale transversale que la section transversale des percées 34, 54, de façon à pouvoir plonger dans les deux percées 34, 54. Ces deux tubes Te et Tc sont conçus de façon que le tube Tc puisse au moins coulisser dans le tube Te, et même aussi y pivoter.

Quand le Praticien veut implanter une telle cage, après l'avoir sortie du blister, il visse l'extrémité du tube Te dans le lamage 36 en maintenant le corps 10 et les pattes 21, 22 par tout moyen, par exemple une pince ou analogue, les pattes étant toujours maintenues dans leur première position P1. Ensuite, il glisse le tube Tc dans le tube Te et dans les deux percées 34, 54.

Il est aussi possible d'introduire d'abord la clef hexagonale centrale Tc dont l'avancement est arrêté par une butée dans la percée 54 de la deuxième patte 22, puis de glisser, sur cette clef, le tube Te qui joue le rôle de préhenseur et qui vient se visser dans le taraudage 38. En fin de vissage, l'embout du tube Te préhenseur vient en appui sur, par exemple, un épaulement de la clef centrale, ce qui emprisonne en pression cette dernière. Ainsi, en finale, la cage et l'ancillaire de pose Ap sont solidement liés pendant l'insertion de la cage entre les vertèbres.

Lorsque la cage est liée à l'ancillaire comme défini ci-dessus, le Praticien peut alors, de façon connue, introduire la cage entre les deux vertèbres jusqu'à ce qu'elle prenne la position voulue par lui. De façon connue, il aura généralement préalablement pris soin de remplir la cavité d'un ou plusieurs greffons osseux ou analogues.

Ensuite, il peut alors agir sur le tube Tc pour faire pivoter les pattes 21, 22 pour qu'elles passent de leur première position P1 à leur seconde position P2 et ainsi s'introduire respectivement dans les deux corps vertébraux des deux vertèbres, comme les pattes ou lames des cages intervertébrales similaires de l'art antérieur.

Il est à noter que la ou les goupilles 64, 164 et les rainures 62, 162 permettent au Praticien de déterminer sans difficulté, et de façon univoque, les deux positions P1 et P2 des pattes, ce qui lui est très pratique, étant donné qu'il n'a pas un accès visuel direct à la cage quand elle est implantée, sauf par radiographie, ce qui permet de lui éviter une utilisation trop prolongée de celle-ci.

Il est aussi possible, pour avoir une plus grande sécurité, que la clef hexagonale soit indexée angulairement, ce qui permet au Praticien de connaître à chaque instant la position des pattes par rapport au corps 10.

Quand cette phase de mise en place de la cage est terminée, il peut agir en force sur les goupilles 64, 164 pour qu'elles s'enfoncent dans leur logement correspondant 71, pour bloquer définitivement les pattes dans leur seconde position P2.

Il peut parfaire ce blocage en introduisant, par exemple, une vis en face avant du corps 10 selon les moyens optionnels 70' décrits ci-avant.

Enfin, en maintenant le tube Tc, le Praticien dévisse le tube Te, puis retire les deux tubes Tc et Te. La cage est alors définitivement implantée.

## Revendications

1. Cage intervertébrale comprenant :
• un corps (10) apte à être interposé entre deux vertèbres, ledit corps comportant deux faces de base (11, 12) opposées sensiblement planes et parallèles et une paroi latérale (13) reliant les deux faces de base,
• au moins une cavité (15) réalisée dans ledit corps (10) en débouchant sur les deux dites faces de base (11, 12),
• une première patte (21), et
• des moyens (30) pour monter la première patte (21) en rotation dans ladite cavité (15) autour d'un axe de rotation (16) sensiblement parallèle au plan des faces de base entre au moins deux première et seconde positions, la première position (P1) étant celle dans laquelle ladite première patte est entièrement contenue dans ladite cavité (15) et la seconde position (P2) étant celle dans laquelle une partie (23) de l'une au moins des extrémités de ladite première patte émerge de ladite cavité, ces dits moyens (30) pour monter en rotation la première patte comportant :
* un premier arbre de rotation (31) solidaire de la première patte (21), et
* un premier orifice traversant cylindrique de révolution (32) réalisé dans ledit corps (10), entre ladite cavité (15) et la paroi latérale (13), et sensiblement centré sur l'axe de rotation (16), le premier arbre de rotation (31) étant monté en rotation dans ce dit premier orifice traversant (32) de façon que son axe soit sensiblement confondu avec ledit axe de rotation (16),
**caractérisée par le fait que** les moyens (30) pour monter en rotation la première patte (21) comportent en outre :
• une première percée traversante cylindrique (34) réalisée dans ledit premier arbre (31) de façon que son axe soit sensiblement confondu avec l'axe de rotation (16), la section transversale (35) de cette première percée traversante (34) étant polygonale.

2. Cage selon la revendication 1, **caractérisée par le fait que** les moyens (30) pour monter en rotation la première patte (21) comportent en outre :
• un lamage (36) réalisé dans l'extrémité (37) du dit premier arbre (31) qui débouche sur la paroi latérale (13) du dit corps (10), ce dit lamage entourant ladite première percée traversante (34) et étant sensiblement centré sur ledit axe de rotation (16), et
• des moyens de fixation (38) d'un outil de préhension réalisés en coopération avec ledit lamage (36).

3. Cage selon l'une des revendications 1 et 2, **caractérisée par le fait que** les moyens de fixation (38) d'un outil de préhension sont constitués d'un taraudage (39) réalisé dans la paroi cylindrique latérale du dit lamage (36).

4. Cage selon l'une des revendications 1 à 3, **caractérisée par le fait qu'**elle comporte en outre :
• une seconde patte (22),
• des moyens (50) pour monter la seconde patte en rotation dans ladite cavité (15) autour du dit axe de rotation (16) entre au moins deux première et seconde positions, la première position étant celle dans laquelle ladite seconde patte est entièrement contenue dans ladite cavité (15) et la seconde position étant celle dans laquelle une partie de l'une au moins des extrémités de ladite seconde patte (22) émerge de la cavité, ces dits moyens (50) pour monter en rotation la seconde patte comportant :
* un second arbre de rotation (51) solidaire de la seconde patte (22), et
* un second orifice traversant cylindrique de révolution (52) réalisé dans ledit corps (10), entre ladite cavité (15) et la paroi latérale (13) du corps, ce second orifice traversant (52) étant sensiblement centré sur ledit axe de rotation (16), le second arbre (51) étant monté en rotation dans ce dit second orifice traversant (52) de façon que son axe soit sensiblement confondu avec ledit axe de rotation, et
* une seconde percée (54) réalisée dans ledit second arbre (51) de façon que son axe soit sensiblement confondu avec l'axe de rotation (16) et qu'elle débouche au moins dans ladite cavité (15), la section transversale de cette seconde percée (54) étant polygonale et ayant une valeur au plus égale à celle de la première percée traversante (34).

5. Cage selon la revendication 4, **caractérisée par le fait que** les sections transversales respectivement des première et seconde percées (34, 54) sont identiques, de préférence de forme polygonale "six pans".

6. Cage selon l'une des revendications précédentes, **caractérisée par le fait qu'**elle comporte en outre des moyens (60) pour limiter l'amplitude de la rotation de ladite première patte (21) par rapport au dit corps (10).

7. Cage selon la revendication 6, **caractérisée par le fait que** les moyens (60) pour limiter l'amplitude de la rotation de ladite première patte (21) par rapport au dit corps (10) comportent :
• au moins une rainure circulaire (62) réalisée dans la paroi extérieure du dit premier arbre (31), ladite rainure circulaire étant inscrite dans un angle (β) défini par les première et seconde positions de ladite première patte,
• un trou (63) percé dans ledit corps (10) et débouchant en regard de ladite rainure circulaire (62) et sur la paroi latérale (13) du dit corps, et
• une goupille (64) disposée dans ledit trou (63) et apte à plonger dans ladite rainure circulaire (62).

8. Cage selon l'une des revendications précédentes, **caractérisée par le fait qu'**elle comporte en outre des moyens (70, 70') pour bloquer ladite première patte (21) quand elle est dans sa seconde position.

9. Cage selon les revendications 7 et 8, **caractérisée par le fait que** les moyens (70) pour bloquer ladite première patte (21) quand elle arrive dans sa seconde position sont constitués par un logement (71) réalisé à l'extrémité de la rainure circulaire (62) située en regard du trou (63) quand ladite première patte (21) est dans sa seconde position, ledit logement (71) étant apte à recevoir l'extrémité pénétrante (72) de la goupille (64) par un enfoncement supplémentaire de celle-ci dans le trou (63).

10. Cage selon l'une des revendications précédentes quand elle dépend de la revendication 4, **caractérisée par le fait qu'**elle comporte des moyens (160) pour limiter l'amplitude de la rotation de ladite seconde patte (22), ces moyens étant constitués par :
• au moins une autre rainure circulaire (162) réalisée dans la paroi extérieure du dit second arbre (51), ladite autre rainure circulaire étant inscrite dans un angle (β') défini par les première et seconde positions de ladite seconde patte (22),
• un autre trou (163) percé dans ledit corps et débouchant en regard de ladite autre rainure circulaire et sur la paroi latérale (13) du dit corps, et
• une autre goupille (164) disposée dans ledit autre trou et apte à plonger dans ladite autre rainure circulaire.

11. Cage selon l'une des revendications précédentes quand elle dépend de la revendication 4, **caractérisée par le fait qu'**elle comporte des moyens pour bloquer ladite seconde patte (22) quand elle arrive dans sa seconde position, ces moyens étant constitués par un autre logement réalisé à l'extrémité de l'autre rainure circulaire située en regard de l'autre trou (163) quand ladite seconde patte est dans sa seconde position, ledit autre logement étant apte à recevoir l'extrémité pénétrante de l'autre goupille (164) par un enfoncement supplémentaire de celle-ci dans l'autre trou (163).

12. Cage selon l'une des revendications précédentes quand elle dépend de la revendication 4, **caractérisée par le fait qu'**elle comporte des moyens pour sertir au moins l'un des deux arbres (31, 51), au niveau de la sortie de l'orifice (32, 52) dans lequel il se trouve, sur la paroi latérale (13) du corps (10) pour fixer l'ensemble patte/arbre sur le corps (10), tout en permettant la rotation de l'arbre dans l'orifice cylindrique de révolution correspondant.

13. Cage selon l'une des revendications précédentes, **caractérisée par le fait que** les moyens (30) pour monter en rotation la première patte (21) comportent en outre des moyens (400) pour la faire pivoter par une de ses deux extrémités.
